# EUROPEAN PATENT SPECIFICATION

(11) **EP 3 585 246 B1**
(45) Date of publication and mention of the grant of the patent: **07.04.2021**
(21) Application number: 18711228.9
(22) Date of filing: 21.02.2018
(51) Int. Cl.: A61B 3/107, A61B 3/14

(54) **COHERENT LASER LIGHT FOR OPTICAL CORNEAL TOPOGRAPHY AND TOMOGRAPHY**
KOHÄRENTES LASERLICHT FÜR OPTISCHE HORNHAUTTOPOGRAFIE UND -TOMOGRAFIE
LUMIÈRE LASER COHÉRENTE POUR TOPOGRAPHIE CORNÉENNE OPTIQUE ET TOMOGRAPHIE

(30) Priority: 23.02.2017 DE 102017202946
(43) Date of publication of application: 01.01.2020
(73) Proprietor: IVIS TECHNOLOGIES S.r.l, 74100 Taranto (IT)
(72) Inventor: D'IPPOLITO, Giuseppe, 74100 Taranto (IT)
(74) Representative: Bardehle Pagenberg Partnerschaft mbB Patentanwälte Rechtsanwälte
(86) International application number: PCT/EP2018/054259
(87) International publication number: WO 2018/153905

(56) References cited:
- US-A1- 2011 149 239
- US-B1- 6 275 718

## Description

### 1. Technical field:

The present invention relates to optical corneal topography and tomography, in particular to the use of coherent laser light for corneal topography and tomography at non-zero angle detection, e.g., using the Scheimpflug principle, and corresponding corneal topographers and tomographers.

### 2. Description of the prior art:

Devices for the evaluation of corneal properties are an indispensable tool in an eye clinic. In ophthalmology, corneal topographers and tomographers are used to determine corneal maps, such as corneal elevation maps, corneal thickness (pachymetry) maps, and refractive power maps that may be used for the diagnosis of refractive disorders and corneal pathologies.

In the prior art, a variety of non-invasive methods have been known for the determination of corneal maps. As an example, devices based on ultrasonic measurements have been used to detect corneal thickness maps. However, their measurements are limited to some points arbitrarily chosen by the operator. In contrast, optical methods for measuring corneal properties typically allow the provision of higher resolution corneal maps. These methods may generally be classified into different groups based on their underlying optical principle, for example, reflection-based techniques and scattering-based techniques may be differentiated.

Reflection is exploited in so-called Placido disk systems. Placido disk-based systems project a series of concentric rings onto the cornea and the associated reflected images are detected. From the deformations in the detected rings, properties of the cornea may be derived. However, if high precision corneal maps are required, these devices are not suitable.

A further technique relying on reflection is the so-called optical coherence tomography. A source beam is split in a reference beam and a measurement beam. The measurement beam is reflected from the cornea and brought into interference with the reference beam to obtain correlation measurements. To allow for such interferometric measurements, sources are used, which provide the required optical coherence, e.g., superluminescent diodes or laser sources. However, due to the required interferometry, optical coherence tomographers are technically highly complex and expensive. In addition, the coaxiality between the source beam and the reflected beam causes parasite reflection, which affects the accuracy of the detected data, especially in the most critical central zone of the cornea.

The most frequently used class of devices for obtaining corneal maps are based on scattering of light. Modern corneal topographers and tomographers of this type typically use a light source, e.g., based on light emitting diodes or incandescence lamps, and project a sharp and bright image of a slit on any desired location of an eye (see, for example LeGrand Y.: "Physiological Optics", New York, Springer, 1980, ISBN 978-3-540-39053-4 or Gills, et al.: "Corneal Topography: The State of the Art, Slack Inc., 1995, ISBN 978-1-556-42268-3). When the slit image is projected onto the cornea, it is scattered by the tissue of the eye. The scattered light is then detected by means of a camera. By arranging the camera in a Scheimpflug configuration and through a triangulation process based on the Scheimpflug principle, a sharp image of a cross section of the cornea may thus be obtained (Merklinger, H.: "Focusing the view camera: A scientific way to focus the view camera and estimate depth of field", 3rd edition, August 1998). Corneal topographers and tomographers using a slit-based system and/or a Scheimpflug configuration are described, e.g., in US 5,512,965 A, WO 01/72213 A1, EP 2 594 192 A1.

Further devices are disclosed in JP H 01-285242 A, EP 1430 829 A1, and US 5 139 022 A

US 6,275,718 discloses an apparatus for determining a corneal topography, comprising a source of a laser beam and a detector positioned at a selected angle of incidence. Devices of the above type relying on scattered light may beneficially be used for determining corneal maps to allow for customized eye surgery. In particular, the precision of laser eye surgery has reached a level that, in principle, allows for extremely precisely customized ablation profiles. However, for fully harnessing this precision, a corresponding precision in the pre-surgical mapping of the individual patient's eye is needed. Therefore, there is a need for improving the performance of corneal topographers and corneal tomographers in delivering corneal maps.

### 3. Summary of the invention:

The above need is at least partly met by an apparatus according to claim 1.

In an example, an apparatus is provided for determining a corneal map. The apparatus comprises a source for providing laser light and the apparatus is adapted to illuminate a portion of a (human) cornea using the laser light. The apparatus further comprises a detector for detecting at least a portion of the laser light being returned from the cornea at a non-zero angle.

It has turned out that - although no interferometric measurements are carried out - the use of laser light in scattering configurations, which detect light returned from the cornea at a non-zero angle, greatly improves the measurement results for the corneal maps. A corneal map may be provided as one or more corneal cross-sections or as a map derived therefrom. The inventors of the present invention have found out that a major reason that limits the accuracy of modern corneal topographers and corneal tomographers are variations in the geometry of the cross section of the light illuminating the cornea. For example, if light having the required intensity and a certain cross section is focused on the center of a cornea (apex), due to the cornea's curvature, the peripheric portions of the cross section will be out of focus, when impinging on the cornea. Thus, the width of, e.g., a slit-shaped cross section of the light that is used for illumination may expand progressively over the impacting surface of the cornea. Due to this defocusing problem, corneal maps are generated, wherein the measurement results are not uniform from the apex to the limbus.

Replacing the light sources commonly used in scattering configurations by a laser source, alleviates this problem: Using a laser source allows an improved focus over the entire illuminated surface of the cornea. Hence, more accurate measurements are facilitated.

A further advantage of using a laser source is that it emphasizes the contrast of the scattering between the epithelium and stroma structure of the cornea. Thus, the use of the laser source also allows the detection of a stromal shape on top of an anterior and a posterior corneal shape, which are detectable with conventional devices based on the detection of scattered light. The detection of the stromal shape allows to measure an epithelium thickness as a difference between the anterior corneal surface and the stromal corneal surface. The obtained epithelium map data allows then for a greater accuracy in the process of customization of corneal surgery.

Particularly using a multimode laser comprising a multimode light emission has turned out to be beneficial to reduce noise and sparkling and to increase homogeneity of the measurements such that edge detection may be enhanced.

The source may provide laser light, e.g. in form of a beam, that propagates along a first axis before impinging on the cornea. The detector is arranged such that it monitors light being returned from the cornea along an optical axis that forms a non-zero angle with the first axis. This angle may be adapted to differ from the angle at which the light propagating along the first axis would be expected to be reflected from the cornea. For example, it may differ by at least 5 degrees, 10 degrees or by at least 25 degrees. Thus, the detected returned light may essentially be returned by scattering. Light intensities provided by the source and used for illuminating the cornea may, e.g., generally be in the range of 0.1 mW/cm² to 20 mW/cm², or in the range of 0.1 mW/cm² to 2 mW/cm².

The apparatus may be configured to provide the laser light such that it comprises a slit-shaped cross section having a long side and a short side. The edges of the cross-section of the laser light may be defined by the contour at which its intensity amounts to half of the maximum intensity of the laser light (e.g. at the center of the slit). For example, the short side may be in the range of 0.01 mm to 1.0 mm, preferably in the range 0.01 mm to 0.1 mm, when impinging on the cornea. This may be achieved using a laser with a corresponding collimator lens and/or further optical elements. This allows to illuminate a desired portion of the cornea sharply with high intensity providing an equally focused image of a slit on the cornea, and still keeping international safety standards for the use of laser light. Hence, a cross-sectional map of the cornea may be obtained. For example, by sequentially illuminating portions of the cornea, a corneal map of essentially the entire cornea may be determined. In other examples, other cross-sectional shapes may be used.

The apparatus may further be configured to provide the laser light such that the slit-shaped cross section, when impinging on the cornea, has a length of at least 6 mm. Preferably, the slit-shaped cross section extends between two opposing limbi, i.e., the cross section, when impinging on the cornea, extends between a point on an edge of the cornea and a point on the opposing edge (approximately 180 degrees) of the cornea. Therefore, to determine corneal maps for a certain area several measurements only need to be carried out along one dimension. Hence, the measurement time is reduced.

The apparatus may be configured to provide the laser light such that a length of the short side is essentially constant when impinging on the cornea, at least it may be constant from the apex of the cornea to the limbus of the cornea. Further, it may also be constant along the entire long side of the slit-shaped cross section when impinging on the cornea. Similarly, also the length of the long side may be essentially constant (along the short side) when impinging on the cornea. Thus, similar illumination properties may be achieved throughout the slit-shaped cross section.

The laser light provides the fundamental advantage that the length of the short side and/or the long side (or, more generally the cross-section of the light used for illumination) may be adapted to be essentially constant in a propagation direction from the apex plane of the cornea to the limbus plane of the cornea. This adaptation may be achieved using laser source optics to collimate the laser light. For example, the use of a laser source, allows limiting a divergence of the light, e.g., to about 1 mrad. With a depth of field of about 3 mm, this implies that the slit diverges at most by about 3 µm when propagating 3 mm. In contrast, light sources used in conventional corneal topographers and tomographers allow at most limiting the divergence to about 1 degree (17.5 mrad), which implies much higher imprecision. Thus, by using a laser source, although the distance between apex plane and limbus plane typically changes between different portions of the cornea due to the pseudo-spherical shape of the cornea, the slit-shaped cross-section of the light used for illuminating the cornea may essentially remain constant. Hence, the illumination properties can be essentially kept constant throughout the different portions of the cornea such that more accurate results may be obtained.

The source of the laser light may, e.g., comprise a diode laser. The inventors found out that the use of a diode laser allows a high-quality edge detection of illuminated corneal tissue such that anterior, stromal, and posterior surfaces of the cornea may reliably be differentiated. In particular, using a diode laser, a more homogeneous scattering from the corneal tissue may be obtained with less noise and/or sparkling. For example, diode lasers operating with wavelengths in the infrared spectrum may be used, e.g., in the range of 700 nm to 1600 nm or from 800 nm to 900 nm. However, to emphasize the contrast level, diode lasers with wavelengths in the blue spectrum, e.g., in the range of 430 nm to 490 nm or 440 nm to 480 nm may be used.

The diode laser may comprise an emission bandwidth, which is larger than 0.2 nm, preferably larger than 0.5 nm, larger than 1 nm or about 2 nm. The diode laser is multimode, i.e., comprise a multimode light emission. For example, the multimode light emission may be over the mentioned emission bandwidths. In other examples, laser sources other than diode lasers may be used that comprise one of the mentioned emission bandwidths and that are multimode. In contrast to the use of laser sources, which are single-mode, and which comprise narrow bandwidths of less than or around 0.1 nm, the use of the above properties has turned out to be beneficial to reduce noise and sparkling and to increase homogeneity of the measurements such that edge detection may be enhanced.

The apparatus may be adapted to provide the laser light with a wavelength in the infrared spectrum, preferably from 700 nm to 1600 nm, or from 800 nm to 900 nm. Also, a wavelength from 430 nm to 490 nm, preferably from 440 nm to 480 nm, or from 440 nm to 460 nm are possible. The inventors found out that, in particular, using the mentioned blue wavelength ranges increases the scattering of the impinging light on the cornea, while at the same time the (visible) light intensities impinging on the patient's eye are still comfortable for the patient. The blue light may be emitted with an intensity in the range of, e.g., 0.1 mW/cm² to 20 mW/cm², which is not only comfortable for the patient but also fulfills international safety standards. Thus, the quality of edge detection will further be increased. It further allows to clearly identify the differences between a corneal epithelium (a relatively thin tissue layer located at the anterior cornea) from a corneal stroma (a relatively thick and transparent middle layer of the cornea).

The apparatus may be configured to determine the corneal map by sequentially illuminating portions of the cornea and detecting the associated portions of the laser light being returned from the cornea at a non-zero angle. The non-zero angles may vary among the measurements regarding different portions. Different maps of the cornea can be determined: A map comprising a full image of the cornea or maps comprising only specific portions of the cornea, as needed. The required positioning of the detector and/or the source may be performed automatically by the apparatus having corresponding motors and control means.

The detector may comprise a sensor and an optical system. The sensor may be arranged to form an angle with the optical system such that the detector is arranged in a Scheimpflug configuration relative to the first axis. The sensor may for example comprise or be implemented by a camera. The optical system may be adapted to focus light onto the sensor. For example, the optical system may comprise an objective or a lens. By using a Scheimpflug configuration, across the entire sensor, a sharp image may be obtained. For example, the optical system can be arranged pivotably and/or rotatably to form an angle with the camera in the range of, e.g., 0 degrees to 90 degrees or 0 degrees to 30 degrees or 0 degrees to 45 degrees. In some examples, additionally or alternatively, the sensor and/or the laser source can be arranged pivotably and/or rotatably. In other examples, the various elements may be arranged in a fixed Scheimpflug configuration without necessarily being pivotable and/or rotatable.

The apparatus may be configured such that the non-zero angle is an acute angle, preferably from 5 degrees to 90 degrees or 10 degrees to 90 degrees.

According a further embodiment, a method is provided for determining a corneal map. A portion of a cornea is illuminated using laser light. At least a portion of the laser light being returned from the cornea is detected at a non-zero angle.

The method may include further steps as outlined in the claims. Generally, the aspects described herein with respect to an apparatus according to the invention may be implemented as a method, and the aspects described herein regarding a method according to the invention may be implemented by the claimed apparatus.

A further embodiment of the present invention relates to a computer program comprising instructions for carrying out the methods described herein. For example, the computer program may be stored on a memory medium and it may cause a processor to implement the method steps. The memory medium and/or the processor may be comprised by an apparatus according to the present invention.

### 4. Brief description of the Figures:

Possible embodiments of the present invention will be described in more detail in the subsequent detailed description with reference to the following figures:
- Fig. 1:: Example of an LED or white light slit source and its projection onto a focusing plane according to the prior art;
- Fig. 2:: Example of an LED or white light slit source and its projection onto a cornea for the use in corneal topography and corneal tomography according to the prior art;
- Fig. 3:: Example of a laser slit source and its projection onto a focusing plane;
- Fig. 4:: Example of a laser slit source and its projection onto a cornea for the use in corneal topography and corneal tomography;
- Fig. 5:: Example of a measurement setup for corneal topography and corneal tomography according to the present invention;
- Fig. 6a:: Example of a corneal cross sectional view obtained with a single mode, narrow bandwidth laser;
- Fig. 6b:: Example of a corneal cross sectional view obtained with an infrared diode laser; and
- Fig. 6c:: Example of a corneal cross sectional view obtained with a blue diode laser.

### 5. Detailed description of possible embodiments:

Possible embodiments of the present invention will be described in the following. For brevity, only a few embodiments can be described. The skilled person will recognize that the specific features described with reference to these embodiments may be modified and combined differently and that individual features may also be omitted if they are not essential. The general explanations in the sections above will also be valid for the following more detailed explanations.

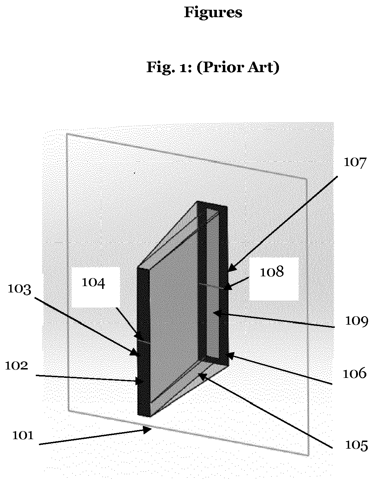

Fig. 1 shows an example for light provided by a light source as used in current corneal topographers and tomographers, e.g., light emitting diodes (LEDs) or white light slit sources according to the prior art. A light having a slit-shaped cross section 102 is focused on a focusing plane 101. The cross section comprises a long side 103 and a short side 104. The light propagates along the direction 105 and the resulting image 106 of the slit on plane 109 also comprises a long side 107 and a short side 108. Due to the defocusing of currently used LEDs or white light sources at the required intensities, the long side 107 and the short side 108 of the projected image of the slit 106 on the focus plane 109 are larger than the long side 103 and the short side 104, respectively, of the source of the slit 102.

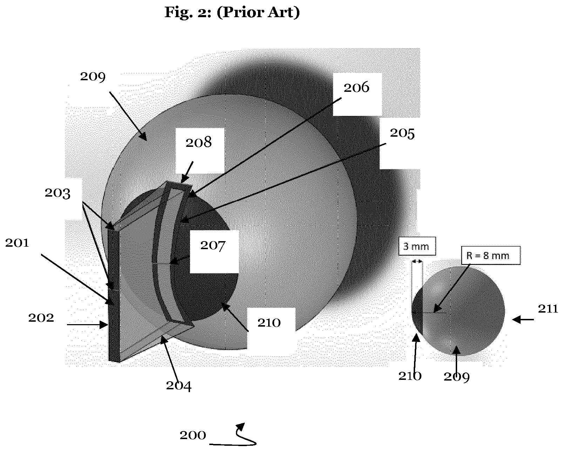

Fig. 2 shows a situation arising in corneal topographers or tomographers 200 according to the prior art. Light having a slit-shaped cross-section 201 comprising a long side 202 and a short side 203 is directed on a cornea 210 of an eye 209. As the light propagates along propagation direction 204, it expands, similarly as explained with reference to Fig. 1. The projected image 205 of the light on a surface of the cornea 210 comprises a long side 206, a first short side 207 at the apex of the cornea, and second short side 208 at the bottom and/or top of the image 205, approximately at a limbus of the cornea 210. As the anterior shape of the cornea 210 is typically similar to a sphere with a radius of approximately 8 mm as shown in the cross-sectional view 211 of the eye 209, and the horizontal distance of the apex to the limbus is typically 3 mm, the first and the second short side, 207 and 208, respectively, of the projected image 205 of the slit differ from each other. The second short side 208 at the limbus is typically unpredictably larger than the first short side 207 at the apex, depending on the distance from the apex plane to the limbus plane, which may vary from case to case. Thus, the more peripheral regions of the cornea will generally be illuminated with light having unpredictably different geometric properties than regions at the center of the cornea, leading to inaccuracy in the detected images.

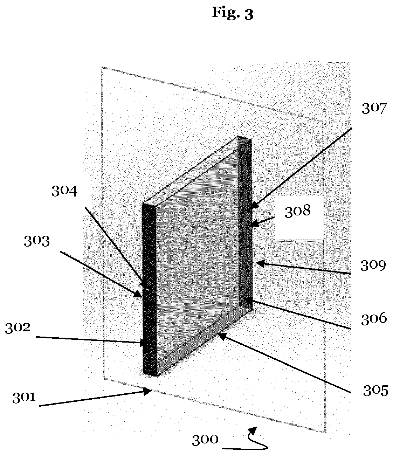

Fig. 3 shows an example for light provided by a coherent light source 300, e.g., provided by a laser. Light having a slit-shaped cross section 302 is projected on focusing plane 301. The cross section comprises a long side 303 and a short side 304. The light propagates along propagation path 305 and the resulting projected image of the slit-shaped cross section 306 on further plane 309 comprises a long side 307 and a short side 308. As coherent light is typically provided by lasers, the long side 307 and the short side 308 of the projected image 306 of the slit on the further plane 309 can typically be provided as large as the long side 303 and the short side 304, respectively, of the source of the slit 302 to much better precision (all other parameters being comparable) than with light sources as explained with reference to Figs. 1 and 2. Therefore, the image of the slit-shaped cross section 306 on the further plane 309 is similar to the slit-shaped cross section 302 as in the focusing plane 301.

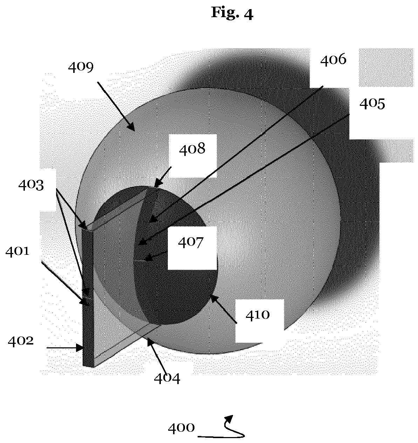

Fig. 4 shows a situation arising in corneal topographers or tomographers 400 when illuminating a cornea according to the present invention. Coherent light having a slit-shaped cross section 401 is provided. For example, a diode laser may emit light with such a cross section, and/or the light emitted by the laser may be shaped accordingly by further optics. Exemplary, a diode laser may be used emitting light with a wavelength in the range of 440 nm to 480 nm, as already explained. Also, diode lasers emitting light in the infrared spectrum may be used. Light may be emitted with intensities in the range of 0.1 mW/cm² to 20 mW/cm², within international safety regulations. In order to form required beams, e.g., comprising a slit-shaped cross section, an additional optical system may be applied, which may include a collimator and/or other optical elements. The cross section comprises a long side 402 and a short side 403 and is projected onto a cornea 410 of an eye 409. In other examples, other cross-sections may be used.

Due to the coherent nature of the light and the typically higher intensities delivered by laser sources, the light can be provided such that its cross-section does not significantly expand in propagation direction 404, which may correspond to a collimated propagation path. The projected image 405 of the slit-shaped cross section on the cornea 410 comprises a long side 406, a first short side 407 at the apex, and second short side 408 at the limbus of the cornea 410. Despite the curvature of the cornea, as explained above with reference to Fig. 2, the first and the second short sides, 407 and 408, respectively, are of essentially the same length. Thus, unpredictable differences of the size of the slit at different regions of the cornea are avoided, and the precision of the measurements is increased.

What is more, the dimensions of the cross section do not significantly change along the collimated propagation path 404. The long side 406 of the image 405 on the cornea 410 is of essentially equal length as the long side 402 of the slit-shaped cross section 401, as it may, e.g. be provided by the source. Additionally, also the short side 403 of the slit-shaped cross section 401, the first short side 407 at the apex of the cornea 410 and the second short side 408 at the limbus of the cornea 410 are also of essentially equal length. Due to this focused image 405 on the cornea 410, a more controlled and a more optimized geometry of the slit across the entire illuminated portions of the cornea is enabled, such that corneal maps with improved precision may be determined.

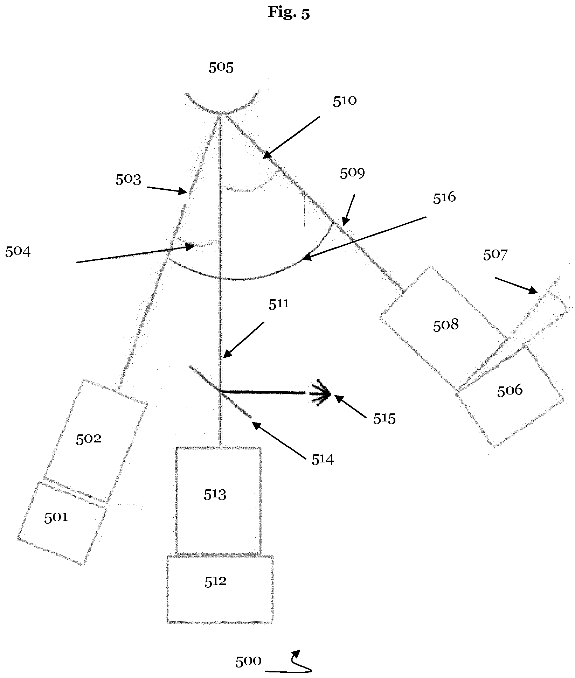

Fig. 5 illustrates a measurement setup 500 for corneal topographers and corneal tomographers according to the present invention. The measurement setup 500 comprises a laser source 501. In non-claimed embodiments, the laser source 501 may be a laser characterized by a single mode emission and/or a narrow emission bandwidth of, e.g., 0.1 nm. Other examples may include diode lasers, characterized by a multimode emission and a wider emission bandwidth, e.g., an emission bandwidth of 2 nm.

Further, the diode laser source may emit light in the infrared spectrum, preferably in the range of 700 nm to 1600 nm or 800 nm to 900 nm or it may emit blue laser light in the wavelength range of 430 nm to 490 nm, preferably 440 nm to 480 nm or 430 nm to 460nm.

The laser source 501 may, optionally, further be equipped with laser source optics 502 to generate laser light, e.g., a light beam that may comprise a slit-shaped cross section, collimated at least for the length of the optical path included between the corneal apex and the corneal limbus, with dimensions of, e.g., 0.01 mm to 1 mm times 6 mm to 10 mm. The emitted laser light may propagate along a first optical axis 503 such that a portion of the cornea 505, e.g., located at a distance in the range of 10 mm to 500 mm, or 50 mm to 200 mm, from the laser source 501 and/or the laser source optics 502, is illuminated. The first optical axis may form a non-zero angle 504 with a central axis 511 of the cornea, preferably in the range of 5 degrees to 45 degrees. The central axis 511 of the cornea may correspond to the visual axis of the patient, which is the direction at which the patient looks at a fixation light generated by a fixation light source 515 and possibly by means of a partially reflecting mirror 514 arranged, e.g., with an angle of 45 degrees relative to the visual axis.

In other examples, no fixation light source 515 and no partially reflecting mirror 514 may be provided. Instead, the central axis 511 of the cornea may be defined as an axis emerging from the illuminated portion of the cornea such that it is perpendicular to the illuminated portion of the cornea.

Laser light is scattered at the cornea 505 and a part of the scattered light propagating along a second optical axis 509 is detected by a detector 506, 508. The detector may be any device capable of detecting scattered light. The detector 506, 508 may comprise a sensor 506, e.g. a camera. For example, an HD CMOS camera may be used. The camera may have a resolution of 2 MPixel to 50 MPixel or 2 MPixel to 5 MPixel. The distance between the camera and the cornea 505 may be in the range of 10 mm to 500 mm or 50 mm to 250 mm. In other examples, another light-sensitive element may be provided instead of camera 506.

Moreover, the detector may optionally comprise an optical system 508. The second optical axis 509 may be defined as the optical axis of the optical system 508. The optical system 508 may shape, e.g. focus, the scattered light before it impinges on the sensor 506. The optical system 508 may provide a magnification by a factor of 0.1 to 10 and provide a field of view of, e.g., 1 mm to 30 mm times 1 mm to 30 mm. The optical system 508 may further be tiltable, e.g. pivotably and/or rotatably, relative to the sensor 506 such that there is an adjustable angle 507 between the sensor 506 and the optical system 508. The optical system 508 and/or the sensor 506 may be pivotably and/or rotatable. The angle 507 may be adjusted between, e.g., 0 degrees to 45 degrees to compensate by a Scheimpflug effect the defocusing of the image generated onto the sensor 506 due to the lack of perpendicularity between the first optical axis 503 and the second optical axis 509 which may form a non-zero angle 516 in the range of, for example, 10 degrees to 90 degrees.

In particular, the angle 507 may be chosen such that the optical axis 503 and the optical axis 509 are coupled to form a Scheimpflug arrangement to produce a sharp image on the sensor 506, hence, leading to a focused image on the entire field of view. Thus, sharp images of portions of the cornea can be obtained across the entire sensor 506. This may be achieved for various angles 516 by appropriately tilting the sensor 506 relative to the optical axis 509. The angle 507 may be calculated as a function of the angle 516 to achieve the Scheimpflug effect. The required positioning of the optical system 508 and the sensor 506 and/or the laser source 501 may be performed automatically by corresponding motors and further control means.

The measurement setup 500 may further comprise, optionally, a registration camera 512 and a registration camera optical system 513. The registration camera 512 may be located, e.g., along the visual axis 511 at a distance, e.g., in the range of 10 mm to 500 mm or 100 mm to 400 mm. The registration camera 512 may, e.g., be a HD CMOS camera providing a resolution of, e.g., 2 MPixel to 50 MPixel or 2 MPixel to 5 MPixel. The registration camera optical system 513 may provide a magnification of, e.g., 0.1 to 10 or 0.5 to 1.5, and provide a field of view of, e.g., 1 mm to 30 mm times 1 mm to 30 mm or 10 mm to 20 mm times 10 mm to 20 mm. The registration camera 512 together with the registration camera optical system 513 may be used to generate a reference image of the cornea 505. A corneal map may be obtained by aligning gathered images of the cornea 505 from the sensor 506 with the reference image of the cornea 505 provided by the registration camera 512. Images from a part of the cornea and/or the whole cornea may be obtained by sequentially illuminating adjacent and/or possibly overlapping portions of the cornea.

Laser source 501, possibly equipped with laser source optics 502, sensor 506 possibly equipped with optical system 508, and the optional registration camera 512 and registration camera optical system 513 may be integrated such as to form part of a corneal tomographer/topographer.

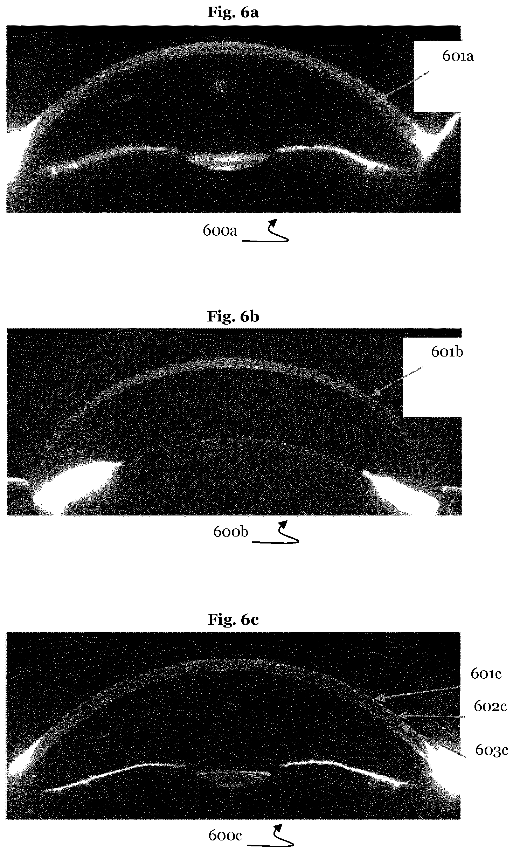

Cross sectional views 600a-c of the detected cross sectional corneal map are shown in Figs. 6a-c. Fig. 6a shows a cross-sectional corneal map 600a, when using a single mode, narrow bandwidth laser light source as characterized above. From the detected cross sectional map the anterior and posterior surfaces of the cornea may be discerned, as well as other elements of the eye. Due to the controlled illumination, the detected maps are highly reliable. Generally, however, some intensity variations will occur within the corneal tissue. Particularly, bright sparks 601a make it difficult to reliably discern the corneal surfaces, e.g., posterior and anterior surface. These slight deficiencies may be ameliorated, as explained below with reference to Figs. 6b and 6c.

From cross-sectional maps detected by the detector, e.g. as shown in Fig. 6a, which may be referred to as "raw" corneal maps, e.g., corresponding to the intensity signal as detected by means of a sensor, further refined maps may be determined. For example, fitting algorithms may be used to extract corneal elevation data, corneal thickness data and/or corneal refractive (or curvature) data. These data, possibly obtained from several "raw" maps as detected by the detector, may be assembled to determine a two or three-dimensional corneal map, e.g. a corneal elevation map, corneal thickness maps and/or corneal refractive (or curvature) map. Such maps may, e.g., indicate the respective elevation, thickness, and/or curvature by a three-dimensional graphical rendering and/or via a color code etc.

A cross sectional view 600b of the detected cross sectional corneal map is shown in Fig. 6b. This map is obtained by using a diode laser emitting light in the infrared spectrum with a wavelength of approximately 840 nm - 860 nm. Generally, wavelengths in the infrared spectrum of 700 nm to 1600 nm may be used. From the obtained cross-sectional corneal map, the anterior and posterior surfaces of the cornea may be discerned, as well as other elements of the eye. Due to the controlled illumination, the detected maps are highly reliable, as explained with reference to Fig. 6a. Moreover, compared to Fig. 6a, the bright sparks 601b are strongly reduced due to the use of the diode laser compared to the single mode, narrow bandwidth lasers as specified above. Thus, the corneal properties may be determined more reliably and across the entire cross section of the cornea.

A cross sectional view 600c of the detected cross sectional corneal map is shown in Fig. 6c. This map is obtained by using a laser source emitting blue laser light with a wavelength of approximately 440 nm to 480 nm, e.g., 450 nm or 460 nm. From the map, the anterior and posterior surfaces of the cornea may be discerned, as well as other elements of the eye, similarly to Figs. 6a and 6b. Compared to Figs. 6a and 6b, however, the cross sectional corneal map 601c shows a higher contrast, which may be attributed to increased scattering of the blue light compared to infrared light. Thus, also a corneal epithelium 602c (a thinner tissue layer located at the anterior cornea) may be distinguished from a corneal stroma 603c (a thicker middle layer of the cornea). Thus, in this example, additional corneal maps may be determined, as the detection of the stromal shape allows to measure the epithelium 602c thickness as a difference between the anterior corneal surface and the stromal corneal surface.

## Claims

1. An apparatus (500) for determining a corneal map (600a, 600b, 600c), comprising:
a. a source (501, 502) for providing laser light, wherein the apparatus (500) is adapted to illuminate a portion of a cornea (505) using the laser light;
b. a detector (506, 508) for detecting at least a portion of the laser light being returned from the cornea (505) at a non-zero angle (516), formed between an axis along which the laser light from the source propagates and an axis along which the at least a portion of the laser light is returned from the cornea;
c. **characterised in that** the source (501, 502) for providing laser light comprises a multimode laser.

2. The apparatus according to claim 1, wherein the apparatus (500) is configured to provide the laser light such that it comprises a slit-shaped cross section (302, 401), the cross-section (302, 401) having a long side (303, 402) and a short side (304, 403).

3. The apparatus according to claim 2, wherein the apparatus (500) is configured to provide the laser light such that the slit-shaped cross section (306, 405), when impinging on the cornea (505), has a length of at least 6 mm, preferably extending between two opposing limbi of the cornea (505).

4. The apparatus according to claim 2 or 3, wherein the apparatus (500) is configured to provide the laser light such that a length of the short side (308, 408) is essentially constant, when impinging on the cornea (505), at least from an apex of the cornea to a limbus of the cornea.

5. The apparatus according to any of claims 2-4, wherein the apparatus (500) is further configured to provide the laser light generated by the source (501) and collimated by laser source optics (502) such that a length of the short side (304, 403) and/or the long side (303, 402) of the laser light is essentially constant in a propagation direction (305, 404) at least from the apex to the limbus.

6. The apparatus according to any of claims 1-5, wherein the multimode laser is a multimode diode laser.

7. The apparatus according to any of claims 1-6, wherein the apparatus (500) is adapted to provide the laser light with a wavelength in the infrared spectrum, preferably from 700 nm to 1600 nm, or with a wavelength from 430 nm to 490 nm, preferably from 440 nm to 480 nm.

8. The apparatus according to any of claims 1-7, wherein the apparatus (500) is configured for sequentially illuminating portions of the cornea (505) and detecting the associated portions of the laser light being returned at a non-zero angle (516) from the cornea (505) for determining the corneal map.

9. The apparatus according to any of claims 1-8, wherein the detector (506, 508) comprises a sensor (506) and an optical system (508), wherein the sensor (506) is arranged to form an angle (507) with the optical system (508) such that the detector is arranged in a Scheimpflug configuration relative to a first axis (503) of the laser light.

10. The apparatus according to any of claims 1-9, wherein the non-zero angle (516) is an acute angle, preferably from 5 degrees to 90 degrees.

11. A method for determining a corneal map (600a, 600b, 600c), wherein the method comprises the following steps:
a. illuminating a portion of a cornea (505) using laser light; and
b. detecting at least a portion of the laser light being returned from the cornea (505) at a non-zero angle (516), formed between an axis along which the laser light from the source propagates and an axis along which the at least a portion of the laser light is returned from the cornea;
c. chracterised in that the laser light is generated by a multimode laser.

12. The method according to claim 11, further comprising adapting the laser light such that it comprises a slit-shaped cross section (302, 401), the cross section having a long side (303, 402) and a short side (304, 403).

13. The method according to claim 12, further comprising adapting the laser light such that the slit-shaped cross section (306, 405), when impinging on the cornea (411), has a length of at least 6 mm, preferably extending between two opposing limbi of the cornea (505).

14. The method according to claim 12 or 13, further comprising adapting the laser light such that a length of the short side (308, 408) is essentially constant, when impinging on the cornea (505), at least from an apex of the cornea to a limbus of the cornea.

15. A computer program comprising instructions which, when executed by a processor, cause the apparatus according to any of claims 1-10 to carry out a method according to any of claims 11-14.

## Patentansprüche

1. Vorrichtung (500) zum Bestimmen einer Hornhautkarte (600a, 600b, 600c), umfassend:
a. eine Quelle (501, 502) zum Bereitstellen von Laserlicht, wobei die Vorrichtung (500) angepasst ist, um einen Teil einer Hornhaut (505) durch Verwenden des Laserlichts zu beleuchten;
b. einen Detektor (506, 508) zum Detektieren mindestens eines Teils des Laserlichts, das von der Hornhaut (505) unter einem Nicht-Null-Winkel (516) zurückgeworfen wird, der zwischen einer Achse, entlang welcher sich das Laserlicht von der Quelle ausbreitet, und einer Achse, entlang welcher der mindestens eine Teil des Laserlichts von der Hornhaut zurückgeworfen wird, ausgebildet ist;
c. **dadurch gekennzeichnet, dass** die Quelle (501, 502) zum Bereitstellen von Laserlicht einen Multimode-Laser umfasst.

2. Vorrichtung nach Anspruch 1, wobei die Vorrichtung (500) konfiguriert ist, um Laserlicht so bereitzustellen, dass es einen schlitzförmigen Querschnitt (302, 401) umfasst, wobei der Querschnitt (302, 401) eine lange Seite (303, 402) und eine kurze Seite (304, 403) aufweist.

3. Vorrichtung nach Anspruch 2, wobei die Vorrichtung (500) konfiguriert ist, um Laserlicht so bereitzustellen, dass der schlitzförmige Querschnitt (306, 405), bei Auftreffen auf die Hornhaut (505), eine Länge von mindestens 6 mm aufweist, sich vorzugsweise erstreckend zwischen zwei gegenüberliegenden Limbi der Hornhaut (505).

4. Vorrichtung nach Anspruch 2 oder 3, wobei die Vorrichtung (500) konfiguriert ist, um Laserlicht so bereitzustellen, dass eine Länge der kurzen Seite (308, 408) bei Auftreffen auf die Hornhaut (505) im Wesentlichen konstant ist, zumindest von einem Scheitelpunkt der Hornhaut zu einem Limbus der Hornhaut.

5. Vorrichtung nach einem der Ansprüche 2-4, wobei die Vorrichtung (500) ferner konfiguriert ist, um das von der Quelle (501) erzeugte und von einer Laserquellenoptik (502) kollimierte Laserlicht so bereitzustellen, dass eine Länge der kurzen Seite (304, 403) und/oder der langen Seite (303, 402) des Laserlichts in einer Ausbreitungsrichtung (305, 404) im Wesentlichen konstant ist, zumindest vom Scheitelpunkt zum Limbus.

6. Vorrichtung nach einem der Ansprüche 1-5, wobei der Multimode-Laser ein Multimode-Diodenlaser ist.

7. Vorrichtung nach einem der Ansprüche 1-6, wobei die Vorrichtung (500) angepasst ist, um das Laserlicht mit einer Wellenlänge im Infrarotspektrum, vorzugsweise von 700 nm bis 1600 nm, oder mit einer Wellenlänge von 430 nm bis 490 nm, vorzugsweise von 440 nm bis 480 nm, bereitzustellen.

8. Vorrichtung nach einem der Ansprüche 1-7, wobei die Vorrichtung (500) konfiguriert ist, um sequenziell Teile der Hornhaut (505) zu beleuchten und die zugehörigen Teile des Laserlichts zu detektieren, die unter einem Nicht-Null-Winkel (516) von der Hornhaut (505) zurückgeworfen werden, um die Hornhautkarte zu bestimmen.

9. Vorrichtung nach einem der Ansprüche 1-8, wobei der Detektor (506, 508) einen Sensor (506) und ein optisches System (508) umfasst, wobei der Sensor (506) angeordnet ist, um mit dem optischen System (508) einen Winkel (507) zu bilden, so dass der Detektor in einer Scheimpflug-Konfiguration relativ zu einer ersten Achse (503) des Laserlichts angeordnet ist.

10. Vorrichtung nach einem der Ansprüche 1-9, wobei der Nicht-Null-Winkel (516) ein spitzer Winkel ist, vorzugsweise von 5 Grad bis 90 Grad.

11. Verfahren zum Bestimmen einer Hornhautkarte (600a, 600b, 600c), wobei das Verfahren die folgenden Schritte umfasst:
a. Beleuchten eines Teils einer Hornhaut (505) unter Verwenden von Laserlicht; und
b. Detektieren mindestens eines Teils des Laserlichts, das von der Hornhaut (505) unter einem Nicht-Null-Winkel (516) zurückgeworfen wird, der zwischen einer Achse, entlang welcher sich das Laserlicht von der Quelle ausbreitet, und einer Achse, entlang welcher der mindestens eine Teil des Laserlichts von der Hornhaut zurückgeworfen wird, ausgebildet ist;
c. **dadurch gekennzeichnet, dass** das Laserlicht von einem Multimode-Laser erzeugt wird.

12. Verfahren nach Anspruch 11, ferner umfassend das Laserlicht so anzupassen, dass es einen schlitzförmigen Querschnitt (302, 401) umfasst, wobei der Querschnitt (302, 401) eine lange Seite (303, 402) und eine kurze Seite (304, 403) aufweist.

13. Verfahren nach Anspruch 12, ferner umfassend das Laserlicht so anzupassen, dass der schlitzförmige Querschnitt (306, 405) bei Auftreffen auf die Hornhaut (411) eine Länge von mindestens 6 mm aufweist, sich vorzugsweise erstreckend zwischen zwei gegenüberliegenden Limbi der Hornhaut (505).

14. Verfahren nach Anspruch 12 oder 13, ferner umfassend das Laserlicht so anzupassen, dass eine Länge der kurzen Seite (308, 408) bei Auftreffen auf die Hornhaut (505) im Wesentlichen konstant ist, zumindest von einem Scheitelpunkt der Hornhaut bis zu einem Limbus der Hornhaut.

15. Ein Computerprogramm umfassend Befehle, welche, wenn sie von einem Prozessor ausgeführt werden, die Vorrichtung nach einem der Ansprüche 1-10 veranlassen, ein Verfahren nach einem der Ansprüche 11-14 auszuführen.

## Revendications

1. Un appareil (500) pour la détermination d'une carte de la cornée (600a, 600b, 600c), comprenant :
a. une source (501, 502) destinée à produire une lumière laser, l'appareil (500) étant apte à illuminer une partie d'une cornée (505) à l'aide de la lumière laser ;
b. un détecteur (506, 508) destiné à détecter au moins une partie de la lumière laser qui est renvoyée par la cornée (505) sous un angle non nul (516) formé entre un axe le long duquel se propage la lumière laser provenant de la source et un axe le long duquel l'au moins une partie de la lumière laser est renvoyée par la cornée ;
c. **caractérisé en ce que** la source (501, 502) destinée à produire une lumière laser comprend un laser multimode.

2. L'appareil selon la revendication 1, dans lequel l'appareil (500) est configuré pour produire une lumière laser de telle manière qu'elle présente une section droite en forme de fente (302, 401), la section droite (302, 401) ayant un côté long (303, 402) et un côté court (304, 403).

3. L'appareil selon la revendication 2, dans lequel l'appareil (500) est configuré pour produire la lumière laser de telle sorte que, lorsqu'elle frappe la cornée (505), la section droite en forme de fente (306, 405) ait une longueur d'au moins 6 mm, de préférence en s'étendant entre deux limbes opposés de la cornée (505).

4. L'appareil selon la revendication 2 ou 3, dans lequel l'appareil (500) est configuré pour produire la lumière laser de telle sorte que, lorsqu'elle frappe la cornée (505), une longueur du côté court (308, 408) soit essentiellement constante au moins d'un sommet de la cornée à un limbe de la cornée.

5. L'appareil selon l'une des revendications 2 à 4, dans lequel l'appareil (500) est en outre configuré pour produire la lumière laser générée par la source (501) et collimatée par une optique de source laser (502) de telle sorte qu'une longueur du côté court (304, 403) et/ou du côté long (303, 402) de la lumière laser soit essentiellement constante dans une direction de propagation (305, 404) au moins du sommet au limbe.

6. L'appareil selon l'une des revendications 2 à 5, dans lequel le laser multimode est un laser multimode à diode.

7. L'appareil selon l'une des revendications 2 à 6, dans lequel l'appareil (500) est apte à produire la lumière laser avec une longueur d'onde dans le spectre infrarouge, de préférence de 700 nm à 1600 nm, ou avec une longueur d'onde de 430 nm à 490 nm, de préférence de 440 nm à 480 nm.

8. L'appareil selon l'une des revendications 2 à 7, dans lequel l'appareil (500) est configuré pour illuminer de façon séquentielle des parties de la cornée (505) et détecter les parties associées de la lumière laser qui est renvoyée sous un angle non nul (516) par la cornée (505) pour déterminer la carte de la cornée.

9. L'appareil selon l'une des revendications 2 à 8, dans lequel le détecteur (506, 508) comprend un capteur (506) et un système optique (508), le capteur (506) étant agencé pour former un angle (507) avec le système optique (508) de telle sorte que le détecteur soit agencé en une configuration de Scheimpflug par rapport à un premier axe (503) de la lumière laser.

10. L'appareil selon l'une des revendications 2 à 9, dans lequel l'angle non nul (516) est un angle aigu, de préférence de 5 degrés à 90 degrés.

11. Un procédé de détermination d'une carte de la cornée (600a, 600b, 600c), le procédé comprenant les étapes suivantes :
a. illumination d'une partie d'une cornée (505) à l'aide d'une lumière laser ; et
b. détection d'au moins une partie de la lumière laser qui est renvoyée par la cornée (505) sous un angle non nul (516) formé entre un axe le long duquel se propage la lumière laser provenant de la source et un axe le long duquel l'au moins une partie de la lumière laser est renvoyée par la cornée ;
c. **caractérisé en ce que** la lumière laser est générée par un laser multimode.

12. Le procédé selon la revendication 11, comprenant en outre l'adaptation de la lumière laser de telle sorte qu'elle présente une section droite en forme de fente (302, 401), la section droite (302, 401) ayant un côté long (303, 402) et un côté court (304, 403).

13. Le procédé selon la revendication 12, comprenant en outre l'adaptation de la lumière laser de telle sorte que, lorsqu'elle frappe la cornée (411), la section droite en forme de fente (306, 405) ait une longueur d'au moins 6 mm, de préférence en s'étendant entre deux limbes opposés de la cornée (505).

14. Le procédé selon la revendication 12 ou 13, comprenant en outre l'adaptation de la lumière laser de telle sorte que, lorsqu'elle frappe la cornée (505), une longueur du côté court (308, 408) soit essentiellement constante au moins d'un sommet de la cornée à un limbe de la cornée.

15. Un programme informatique comprenant des instructions qui, lorsqu'elles sont exécutées par un processeur, font en sorte que l'appareil selon l'une des revendications 1 à 10 mette en œuvre un procédé selon l'une des revendications 11 à 14.
